(19) **Europäisches Patentamt / European Patent Office / Office européen des brevets**

(11) **EP 4 567 122 A1**

(12) **EUROPEAN PATENT APPLICATION**
published in accordance with Art. 153(4) EPC

(43) Date of publication:
**11.06.2025 Bulletin 2025/24**

(21) Application number: **23850430.2**

(22) Date of filing: **02.08.2023**

(51) International Patent Classification (IPC):
*C12P 7/625* (2022.01)   *C12N 1/20* (2006.01)

(52) Cooperative Patent Classification (CPC):
**C12N 1/20; C12P 7/625**

(86) International application number:
**PCT/KR2023/011343**

(87) International publication number:
**WO 2024/029930 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.08.2022 KR 20220097461**

(71) Applicant: **CJ CheilJedang Corporation**
**Seoul 04560 (KR)**

(72) Inventors:
• **KIM, Da Eun**
**Seoul 04560 (KR)**

• **SEO, Dong June**
**Seoul 04560 (KR)**
• **LEE, Han-Sol**
**Seoul 04560 (KR)**
• **LEE, Ilgyu**
**Seoul 04560 (KR)**
• **KIM, Jung Min**
**Seoul 04560 (KR)**
• **HONG, Chang Young**
**Seoul 04560 (KR)**

(74) Representative: **Meissner Bolte Partnerschaft mbB**
**Patentanwälte Rechtsanwälte**
**Postfach 86 06 24**
**81633 München (DE)**

(54) **METHOD AND SYSTEM FOR CONTROLLING PURIFICATION PROCESS THROUGH COLOR VALUE MONITORING, AND METHOD FOR PRODUCING POLYHYDROXYALKANOATE RESIN**

(57)   According to the present invention, a high-quality polyhydroxyalkanoate resin having excellent color quality can be reliably produced by monitoring the color value during the process of purifying a crude solution containing polyhydroxyalkanoate (PHA) and controlling the conditions of a purification step in real time on the basis thereof.

[Fig. 1]

EP 4 567 122 A1

## Description

### Technical Field

The present disclosure relates to a method and a system for controlling a purification process for preparing a polyhydroxyalkanoate resin through monitoring of a color value in the purification process. In addition, the present disclosure relates to a process for preparing a polyhydroxyalkanoate resin with excellent color quality using the same.

### Background Art

Polyhydroxyalkanoates (PHAs) are biodegradable polymers composed of several types of hydroxyl carboxylic acids produced by numerous microorganisms and used as intracellular storage materials. Polyhydroxyalkanoates have physical properties similar to those of conventional petroleum-derived synthetic polymers such as polybutylene adipate terephthalate (PBAT), polybutylene succinate (PBS), polybutylene succinate terephthalate (PBST), and polybutylene succinate adipate (PBSA), exhibit complete biodegradability, and are excellent in biocompatibility.

Specifically, the polyhydroxyalkanoate is a natural thermoplastic polyester polymer that accumulates in microbial cells. Since it is a biodegradable material, it can be composted and finally decomposed into carbon dioxide, water, and organic waste without generating toxic waste. In particular, the polyhydroxyalkanoate is biodegradable under any environmental conditions, such as soil and sea, and has environmentally friendly characteristics.

In a common process for preparing a polyhydroxyalkanoate resin, PHA particles formed and accumulated within microbial cells are surrounded by proteins, phospholipids, polypeptides, or the like even after extraction. Thus, additional purification processes such as decolorization and deproteinization are carried out to remove these impurities.

[Prior Art Document]

(Patent Document 1) Korean Laid-open Patent Publication No. 2001-0009719

### Disclosure of Invention

### Technical Problem

The impurities to be removed through decolorization and deproteinization in a process for preparing a polyhydroxyalkanoate resin are of various types, including cell-derived components. There has been a problem in that it is not possible to distinguish and qualitatively or quantitatively determine the impurities present in solid PHA particles and a liquid in real time while the process is carried out. In particular, as impurities remaining in PHA resin particles cause coloration and deterioration of polymer properties during processing, they must be strictly managed in the mass production process. However, real-time management of the process for impurity removal has been very difficult due to the problems described above.

As a result of research conducted by the present inventors, it is possible to devise a process for stably preparing a polyhydroxyalkanoate resin with excellent color quality by monitoring a color value of a process solution during the purification process and adjusting the purification conditions such as decolorization based on the color value or predicting the color value of a final product.

An object of the present disclosure is to provide a method and a system for controlling a purification process through monitoring of a color value in the purification process and a process for preparing a polyhydroxyalkanoate resin with excellent color quality using the same.

### Solution to Problem

According to an aspect of the present disclosure, there is provided a method for controlling a purification process, which comprises measuring a color value of a process solution or its supernatant in the purification process for preparing a polyhydroxyalkanoate (PHA) resin, or a color value of a purified solution and its supernatant after the purification process, and controlling the purification process based on the measured color value.

In an embodiment, the measurement of the color value comprises measuring an a* value in the CIE LAB color space, and the purification process conditions may be adjusted based on the change in the a* value of the process solution or its supernatant.

In another embodiment, the change in the a* value may be confirmed by comparing the a* value of the process solution or its supernatant with the a* value of a crude solution or its supernatant before the purification process, or by the change in the a* value of the process solution or its supernatant over time during the purification process.

In another embodiment, the measurement of the color value comprises measuring an a* value in the CIE LAB color space, and the b* value of a polyhydroxyalkanoate resin in the CIE LAB color space may be predicted based on the a* value of the purified solution and its supernatant.

According to another aspect of the present disclosure, there is provided a system for controlling a purification process, which comprises a purification unit for carrying out the purification process for preparing a polyhydroxyalkanoate (PHA) resin; a monitoring unit for measuring a color value of a process solution or its supernatant during the purification process, or a color value of a purified solution and its supernatant after the purification process, and a control unit for controlling the purification process based on the measured color value.

According to another aspect of the present disclosure, there is provided a process for preparing a polyhydroxyalkanoate (PHA) resin from a crude solution comprising a polyhydroxyalkanoate through a purification process, which comprises at least one step among (a) measuring a color value of a process solution or its supernatant during the purification process; and (b) measuring a color value of a purified solution and its supernatant after the purification process.

In an embodiment, the measurement of the color value in step (a) comprises measuring the a* value of the process solution or its supernatant in the CIE LAB color space, and the purification process conditions may be adjusted based on the change in the a* value of the process solution or its supernatant.

In another embodiment, the process further comprises measuring the a* value of the crude solution or its supernatant, and the change in the a* value of the process solution or its supernatant relative to the a* value of the crude solution or its supernatant may be confirmed.

In another embodiment, the purification process comprises a decolorization process, wherein the decolorization process is carried out by using a first oxidizing agent, a second oxidizing agent, or both in sequence, the first oxidizing agent may comprise sodium chlorite ($NaClO_2$), and the second oxidizing agent may comprise hydrogen peroxide ($H_2O_2$).

In another embodiment, the decolorization process is carried out by the second oxidizing agent, and the amount of the oxidizing agent added to the decolorization process may be increased when the following Relationship (1A) is satisfied.

$$a0 < a30 \qquad (1A)$$

In Relationship (1A), a0 is the a* value of the crude solution, and a30 is the a* value of the process solution after the addition of the second oxidizing agent.

In another embodiment, the decolorization process is carried out by using the first oxidizing agent and the second oxidizing agent in sequence, and the amount of at least one of the first oxidizing agent and the second oxidizing agent added to the decolorization process may be increased when the following Relationship (1B) is satisfied.

$$a0' > a30' \qquad (1B)$$

In Relationship (1B), a0' is the a* value of the supernatant of the crude solution, and a30' is the a* value of the supernatant of the process solution after the addition of the first oxidizing agent.

In an embodiment, the measurement of the color value in step (b) comprises measuring the a* value of the purified solution and its supernatant, and the b* value of the polyhydroxyalkanoate resin in the CIE LAB color space may be predicted based on the a* value of the purified solution and its supernatant.

In another embodiment, the b* value of the polyhydroxyalkanoate resin may be predicted from the following Relationship (2A).

$$b* = Y + (X1 \times a1) + (X2 \times a2) \qquad (2A)$$

In Relationship (2A), a1 is the a* value of the purified solution, a2 is the a* value of the supernatant of the purified solution, Y is 10 to 21, X1 is -2.5 to 4.0, and X2 is -3.5 to 3.0. More specifically, Y is 14.68 to 16.68, X1 is -0.39 to 1.61, and X2 is -1.35 to 0.65.

In another embodiment, the b* value of the polyhydroxyalkanoate resin may be predicted from the following Relationship (2B).

$$b* = Y' + (X1' \times a1') + (X2' \times a2') \qquad (2B)$$

In Relationship (2B), a1' is the a* value of the purified solution, a2' is the a* value of the supernatant of the purified solution, Y' is 4 to 15, X1' is -7 to -1, and X2' is -8 to -1. More specifically, Y' is 8.15 to 10.15, X1' is -5.15 to -3.15, and X2' is -5.04 to -3.04.

In another embodiment, if the predicted b* value of the polyhydroxyalkanoate resin is greater than the target b* value, an additional purification process may be carried out.

In another embodiment, the crude solution comprising the polyhydroxyalkanoate may be prepared by a method comprising performing a solid-liquid separation of a fermentation broth comprising a polyhydroxyalkanoate to obtain biomass; mixing an additive comprising a surfactant with the biomass to obtain a suspension; crushing the biomass in the suspension to obtain a slurry; and performing a solid-liquid separation of the slurry.

**Advantageous Effects of Invention**

According to the method and system of the present disclosure, the color value is monitored during the purification process, and the purification process conditions can be adjusted based on the color value to remove impurities more effectively, and the color value of a final product can be predicted and responded to in advance. Accordingly, a polyhydroxyalkanoate resin with excellent color quality can be stably prepared.

**Brief Description of Drawings**

Fig. 1 illustrates a method for controlling a purification process through monitoring of a color value.

Fig. 2a illustrates a process for preparing a PHA resin comprising a purification process (using only $H_2O_2$ for decolorization) according to an embodiment.

Fig. 2b illustrates a process for preparing a PHA resin comprising a purification process (using $NaClO_2$ and $H_2O_2$ for decolorization) according to another embodiment.

Fig. 3a shows the results of monitoring the color value over time in the purification process in the process for preparing a PHA resin according to an embodiment.

Fig. 3b shows the results of monitoring the color value over time in the purification process in the process for preparing a PHA resin according to another embodiment.

Fig. 4a shows the correlation between the predicted b* value and the actually measured b* value of a PHA resin in the process for preparing the PHA resin according to an embodiment.

Fig. 4b shows the correlation between the predicted b* value and the actually measured b* value of a PHA resin in the process for preparing the PHA resin according to another embodiment.

Fig. 5 schematically illustrates the concept of predicting a color value of a final PHA resin assumed in the present disclosure.

Fig. 6 shows the concepts of L*, a*, and b* of the CIE (Commission Internationale de l'Eclairage) LAB color space.

**Best Mode for Carrying out the Invention**

Hereinafter, the present disclosure will be described in more detail with reference to various embodiments.

In this specification, terms referring to the respective components are used to distinguish them from each other and are not intended to limit the scope of the present disclosure. In addition, in the present specification, a singular expression is interpreted to cover a plural number as well unless otherwise specified in the context.

In the present specification, the term "comprising" is intended to specify a particular characteristic, region, step, process, element, and/or component. It does not exclude the presence or addition of any other characteristic, region, step, process, element and/or component, unless specifically stated to the contrary.

In the present specification, the terms first, second, and the like are used to describe various components. But the components should not be limited by the terms. The terms are used for the purpose of distinguishing one element from another.

**Control of a purification process through monitoring a color value and preparation of a PHA resin**

An aspect of the present disclosure provides a method for controlling a purification process of a polyhydroxyalkanoate (PHA) resin through monitoring of a color value. The method for controlling a purification process comprises measuring a color value of a process solution or its supernatant in the purification process for preparing a polyhydroxyalkanoate (PHA) resin, or a color value of a purified solution and its supernatant after the purification process, and controlling the purification process based on the measured color value.

The process solution may be obtained in a procedure of carrying out a purification process (e.g., a decolorization process or a deproteinization process) of a crude PHA solution, and the purified solution may be obtained upon completion of the purification process. The crude solution, the process solution, and the purified solution may each comprise a PHA, impurities, a solvent, and the like and may have a form such as a solution, a suspension, a dispersion, a slurry, or the like. In addition, the color value may be measured by separating a supernatant from the crude solution, the process solution, or the purified solution. The supernatant refers to the upper liquid excluding insoluble solids that have settled to the bottom through centrifugation, sedimentation, or the like.

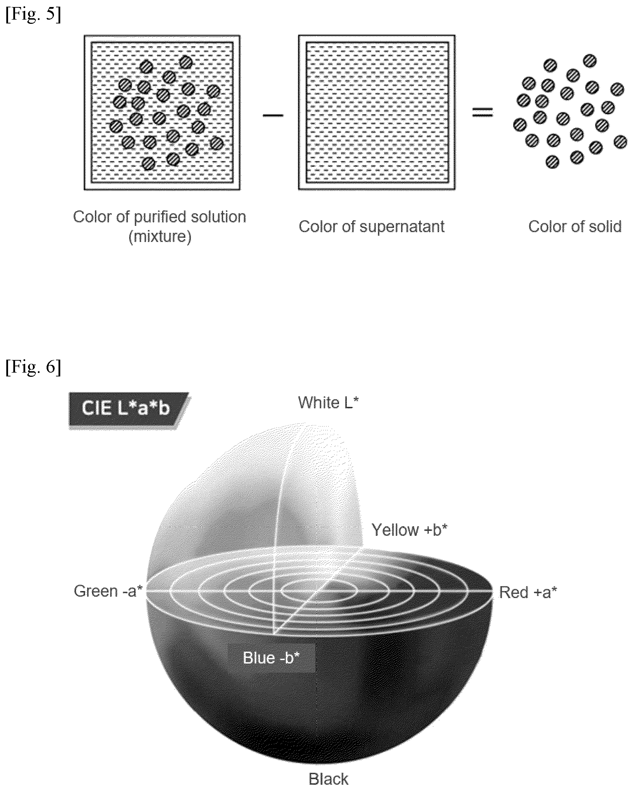

As described above, the present disclosure comprises monitoring of a color value during the process, which method has the advantages that no separate preliminary treatment of a sample to be analyzed is required and that rapid analysis and immediate confirmation of results are possible. The color value monitored in the present disclosure includes a* and b* in the CIE LAB color space, which can be measured using a spectrophotometer. Fig. 6 shows the concepts of L*, a*, and b* of the CIE LAB color space (source: https://terms.naver.com/entry.naver?docId=1118148&cid=40942&cate goryId=33048). Referring to Fig. 6, the a* value indicates whether the color is closer to red or green. If it is negative, it is closer to green. If it is positive, it is closer to red. The b* value indicates whether the color is closer to blue or yellow. If it is negative, it is closer to blue. If it is positive, it is closer to yellow.

The impurities to be removed through the purification process in the process for preparing a polyhydroxyalkanoate resin are of various types, including cell-derived components. There has been a problem in that it is not possible to distinguish and qualitatively or quantitatively determine the impurities present in solid PHA particles and a liquid in real time during the purification process. In particular, as impurities remaining in PHA resin particles cause coloration and deterioration of polymer properties during processing, they must be strictly managed in the mass production process. However, real-time management of the process for impurity removal has been very difficult due to the problems described above.

The present inventors have noted that since the b* value of a final PHA resin increases as the amount of impurities increases, the b* value reflects the content of impurities in the final PHA resin to some extent. Accordingly, the present inventors have analyzed data obtained by monitoring color values in several purification processes to find that the a* value measured during the purification process has the most significant relationship with the b* value of a final PHA resin (see Table 1).

As a result, the measurement of a color value in the present disclosure may comprise measuring the a* value in the CIE LAB color space.

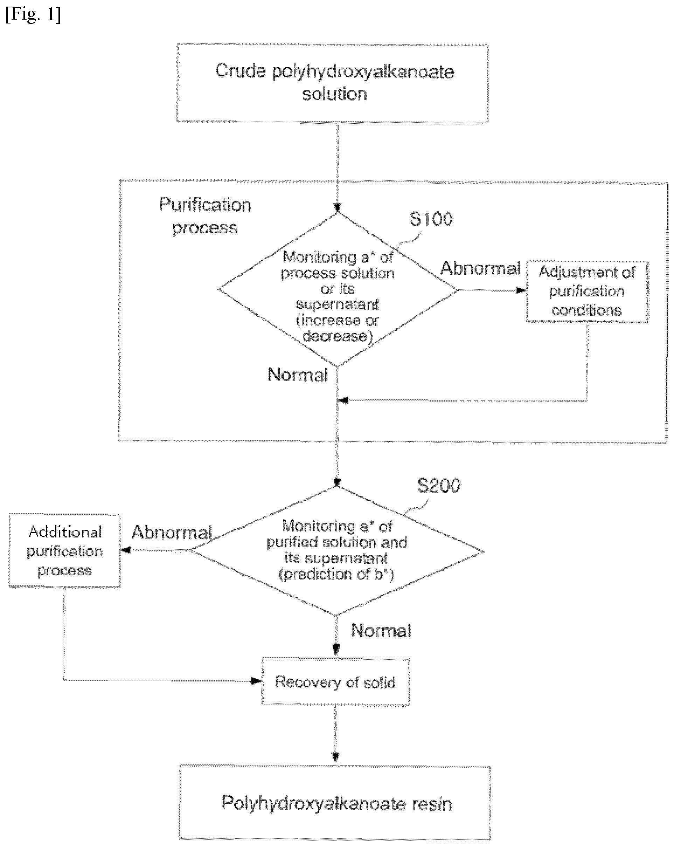

Fig. 1 illustrates a method for controlling a purification process through monitoring of a color value. Referring to Fig. 1, the method comprises monitoring the a* value of the process solution or its supernatant in the CIE LAB color space during the purification process (S100) and/or monitoring the a* value of the purified solution and its supernatant after the purification process (S200).

According to an embodiment, the purification process conditions may be adjusted based on the change in the a* value of the process solution or its supernatant.

For example, the change in the a* value may be confirmed by comparing the a* value of the process solution or its supernatant with the a* value of the crude solution or its supernatant before the purification process, or by the change in the a* value of the process solution or its supernatant over time during the purification process.

As a specific example, the change in the a* value of the process solution or its supernatant is monitored at the beginning of the purification process to distinguish normal and abnormal states based on the increase or decrease thereof. If it is abnormal, the amount of an oxidizing agent added to the decolorization process can be increased. For this purpose, the process further comprises measuring the a* value of the crude solution or its supernatant, and the change in the a* value of the process solution or its supernatant relative to the a* value of the crude solution or its supernatant may be confirmed.

As another specific example, the change in the a* value of the process solution or its supernatant is continuously monitored while the purification process is carried out to distinguish normal and abnormal states based on the increase or decrease thereof. If it is abnormal, the amount of an oxidizing agent added to the decolorization process can be increased. For this purpose, after the purification process has been initiated, the a* value of the process solution or its supernatant is repeatedly measured at regular time intervals, for example, at 10-minute intervals, 20-minute intervals, or 30-minute intervals. These a* values may be compared to determine whether there is a decrease or an increase.

According to another embodiment, the b* value of a polyhydroxyalkanoate resin in the CIE LAB color space may be predicted based on the a* value of the purified solution and its supernatant.

Another aspect of the present disclosure provides a system for controlling a purification process of a polyhydroxyalkanoate (PHA) resin through monitoring of a color value. The system for controlling a purification process comprises a purification unit for carrying out the purification process for preparing a polyhydroxyalkanoate (PHA) resin; a monitoring unit for measuring a color value of a process solution or its supernatant during the purification process, or a color value of a purified solution and its supernatant after the purification process, and a control unit for controlling the purification process based on the measured color value.

The purification unit may comprise, for example, a decolorization unit and a deproteinization unit. A crude PHA solution is fed to the decolorization unit and decolorized, and the process solution is then passed through the deproteinization unit to provide a purified solution whose deproteinization has been completed. In addition, an acid or alkaline component for pH adjustment and one or more types of oxidizing agents for decolorization may be added to the decolorization unit. Decolorization may be carried out at least once. An acid or alkaline component for pH adjustment and one or more types of proteases for deproteinization may be added to the deproteinization unit. Accordingly, the decolorization unit and the deproteinization unit are each provided with a reactor, and the reactor may be equipped with an inlet for feeding raw materials and samples, an outlet for discharging a process solution or a purified solution, and a temperature controller. A connecting unit such as a pipe for connecting the inlet and outlet between the reactors may be equipped.

The monitoring unit may comprise, for example, a spectrophotometer capable of measuring a color value of a sample. In addition, the monitoring unit may comprise an extractor for extracting a sample of a portion of the crude solution, the process solution, or the purified solution from each reactor provided in the purification unit. It may also comprise a centrifuge for separating a supernatant from a sample of the process solution or the purified solution.

The control unit may be provided with an analyzer that receives information on the color value measured by the monitoring unit and analyzes it (comparison, interpretation, determination, prediction, and the like). For transmitting such color value information, the control unit and the monitoring unit may be electrically connected. In addition, the control unit may be further provided with a processor that adjusts the purification process conditions or transmits feedback on whether to carry out an additional purification process based on the results analyzed by the analyzer. The control unit is electrically connected to the purification unit as well such that the flow of the purification process can be controlled according to the feedback transmitted from the processor.

According to the method and system of the present disclosure, the results of monitoring color values during the process are applied to the purification process in real time, whereby impurities can be removed more effectively. As the color value of a final PHA resin can be predicted and responded to in advance, a PHA resin having excellent color quality can be stably prepared.

Another aspect of the present disclosure provides a process for preparing a polyhydroxyalkanoate resin through monitoring of a color value. The process for preparing a polyhydroxyalkanoate (PHA) resin from a crude solution through a purification process comprising a polyhydroxyalkanoate, which comprises at least one step among (a) measuring a color value of a process solution or its supernatant during the purification process; and (b) measuring a color value of a purified solution and its supernatant after the purification process.

In the process of the present disclosure, the purification process may comprise, for example, a decolorization process and a deproteinization process.

Hereinafter, each process and step of the preparation process according to the present disclosure will be described in detail.

**Polyhydroxyalkanoate**

Polyhydroxyalkanoate (PHA) may be classified as crystalline, semi-crystalline, or amorphous polyhydroxyalkanoate, depending on its molecular structure.

According to an embodiment, the polyhydroxyalkanoate may comprise an amorphous polyhydroxyalkanoate. In the case of an amorphous polyhydroxyalkanoate, repeated solid-liquid separation and washing are difficult during the water-based purification process due to its unique flexibility and tackiness, whereby the content of impurities in a purified final PHA is still high, making difficult the commercialize thereof. According to the present disclosure, however, as decolorization using two types of oxidizing agents is sequentially carried out during the purification process of an amorphous PHA, the color of a final product can be significantly improved.

According to another embodiment, the polyhydroxyalkanoate may comprise a semi-crystalline polyhydroxyalkanoate. According to another embodiment, the polyhydroxyalkanoate may comprise a crystalline polyhydroxyalkanoate. According to another embodiment, the polyhydroxyalkanoate may comprise two or more of an amorphous polyhydroxyalkanoate, a semi-crystalline polyhydroxyalkanoate, and a crystalline polyhydroxyalkanoate.

The polyhydroxyalkanoate may comprise at least one repeat unit selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxybutyrate (4-HB), 4-hydroxyvalerate (4-HV), 5-hydroxyvalerate (5-HV), and 6-hydroxyhexanoate (6-HH).

In addition, the polyhydroxyalkanoate may comprise isomers. For example, the polyhydroxyalkanoate may comprise structural isomers, enantiomers, or geometric isomers. Specifically, the polyhydroxyalkanoate may comprise structural isomers.

The polyhydroxyalkanoate may be a homopolymer or a copolymer. According to an embodiment, the polyhydroxyalkanoate may comprise a copolymer, specifically, a copolymer comprising two or more different repeat units with the different repeat units randomly distributed in the polymer chain.

As an example, the polyhydroxyalkanoate may comprise a 4-hydroxybutyrate (4-HB) repeat unit. For example, the content of a 4-hydroxybutyrate (4-HB) repeat unit in the polyhydroxyalkanoate may be 0.1% by weight or more, 1% by weight or more, 5% by weight or more, 10% by weight or more, 20% by weight or more, 30% by weight or more, 40% by weight or more, 50% by weight or more, or 60% by weight or more, and 100% by weight or less, 99% by weight or less, 90% by weight or less, 80% by weight or less, 70% by weight or less, or 60% by weight or less, based on the total weight of the polyhydroxyalkanoate. Specifically, the polyhydroxyalkanoate may comprise a 4-hydroxybutyrate (4-HB) repeat unit in an amount of 25% by weight or more.

Specifically, the polyhydroxyalkanoate may be a copolymer comprising a 4-HB repeat unit. Examples of the repeat units that may be contained in the polyhydroxyalkanoate, together with 4-HB, may include at least one selected from the group consisting of lactic acid, glycolic acid, 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

More specifically, the polyhydroxyalkanoate may comprise one or more repeat units selected from the group consisting of 3-HB, 3-HP, 3-HH, 3-HV, 4-HV, 5-HV, and 6-HH, in addition to a 4-HB repeat unit. As an example, the polyhydroxyalkanoate may comprise a copolymer comprising a 4-HB repeat unit and a 3-HB repeat unit. Specifically, it may comprise poly-3-hydroxybutyrate-co-4-hydroxybutyrate (P3HB-co-4HB).

The PHA may comprise one or more types of PHAs. For example, it may comprise a first PHA, a second PHA, or a mixture thereof. For example, the first PHA may be an amorphous PHA, and the second PHA may be a semi-crystalline PHA. The first PHA may comprise a 4-HB repeat unit in an amount of, for example, 15% by weight to 60% by weight, 15% by weight to 55% by weight, 20% by weight to 55% by weight, 25% by weight to 55% by weight, 30% by weight to 55% by weight, 35% by weight to 55% by weight, 20% by weight to 50% by weight, 25% by weight to 50% by weight, 30% by weight to 50% by weight, 35% by weight to 50% by weight, or 20% by weight to 40% by weight. The second PHA may comprise a 4-HB repeat unit in an amount of, for example, 0.1% by weight to 30% by weight, 0.5% by weight to 30% by weight, 1% by weight to 30% by weight, 3% by weight to 30% by weight, 1% by weight to 28% by weight, 1% by weight to 25% by weight, 1% by weight to 24% by weight, 1% by weight to 20% by weight, 1% by weight to 15% by weight, 2% by weight to 25% by weight, 3% by weight to 25% by weight, 3% by weight to 24% by weight, 5% by weight to 24% by weight, 5% by weight to 20% by weight, greater than 5% by weight to less than 20% by weight, 7% by weight to 20% by weight, 10% by weight to 20% by weight, 15% by weight to 25% by weight, or 15% by weight to 24% by weight. In addition, the PHA may be a mixture of the first PHA and the second PHA at a weight ratio of 20:80 to 80:20 or 30:70 to 70:30.

**Crude polyhydroxyalkanoate solution**

The crude polyhydroxyalkanoate solution may be prepared by a fermentation process.

The method for producing a polyhydroxyalkanoate (PHA) through fermentation may be carried out by a known method. For example, a polyhydroxyalkanoate can be produced through fermentation using wild-type or transgenic microorganisms cultured on a specific substrate, and it is not particularly limited.

Upon fermentation, the polyhydroxyalkanoate thus produced accumulates within microbial cells; thus, a process of separating and purifying the same is absolutely necessary.

For example, biomass containing a polyhydroxyalkanoate is separated from the fermentation broth, a surfactant is added thereto, and the microbial cells are crushed to separate the polyhydroxyalkanoate only.

As an example, the crude solution comprising a polyhydroxyalkanoate may be prepared by a method comprising performing a solid-liquid separation of a fermentation broth comprising a polyhydroxyalkanoate (PHA) to obtain biomass; mixing an additive comprising a surfactant with the biomass to obtain a suspension; crushing the biomass in the suspension to obtain a slurry; and performing a solid-liquid separation of the slurry.

First, a fermentation broth formed by microorganisms is subjected to a solid-liquid separation using a mechanical separation method such as centrifugation or membrane separation or other known methods to separate biomass comprising cells in which a polyhydroxyalkanoate (PHA) has accumulated.

The biomass thus separated may be mixed with a solvent to adjust the solids concentration. In addition, the biomass may be mixed with a surfactant. For example, an anionic surfactant or a nonionic surfactant may be used as the surfactant. Specifically, sodium laureth sulfate, sodium lauryl sulfate, dodecyl sodium sulfate, and dodecyl sodium benzene sulfonate may be used. As a result, a suspension comprising biomass, surfactant, or the like may be obtained.

Thereafter, the biomass in the suspension is crushed, and the crushing may be carried out by mechanical crushing (physical crushing) or chemical crushing (nonmechanical crushing). For example, the crushing may be carried out by ultrasonic crushing, high-pressure crushing, or milling crushing. The ultrasonic crushing may be carried out, for example, for 10 minutes to 60 minutes at an energy level of 20 Hz or higher. In addition, the high-pressure crushing may be carried out, for example, for 1 minute to 60 minutes at a pressure of 10 bar or more. In addition, the milling crushing may be carried out for 1 minute to 60 minutes by a colloid mill, a bead mill, or a ball mill.

As a result of crushing, a slurry comprising fine particles of a polyhydroxyalkanoate and cell-derived impurities such as cell walls may be obtained. The slurry is subjected to a solid-liquid separation using a mechanical separation method such as centrifugation or membrane separation or other known methods to obtain a crude polyhydroxyalkanoate solution.

Since the crude solution of a polyhydroxyalkanoate comprises trace amounts of cell-derived impurities (proteins, lipids, and the like), it requires further purification.

For example, the content of proteins in the crude solution may be 3.5% by weight or less, 3% by weight or less, 2.7% by weight or less, or 2.5% by weight or less, and may be 0.1% by weight or more, 1% by weight or more, or 1.5% by weight or more, based on the total solids weight in the crude solution.

In addition, the content of lipids in the crude solution may be 1.5% by weight or less, 1.3% by weight or less, 1% by weight or less, or 0.7% by weight or less, and may be 0.1% by weight or more, 0.2% by weight or more, or 0.3% by weight or more, based on the total solids weight in the crude solution.

In addition, the solids content in the crude solution may be 1% by weight or more, 3% by weight or more, 5% by weight or more, 7% by weight or more, or 10% by weight or more, and may be 80% by weight or less, 50% by weight or less, 30% by weight or less, 20% by weight or less, or 15% by weight or less, based on the total weight of the crude solution.

In addition, the supernatant may be separated from the crude solution thus prepared by centrifugation, sedimentation, or the like to be used for the measurement of a color value, which will be explained in detail in the section of monitoring of a color value below.

**Purification process**

The purification process for preparing a PHA resin from the crude solution may comprise a decolorization process.

The decolorization process may be carried out by adding one or more types of oxidizing agents to the crude solution. Specifically, the decolorization process is carried out by using a first oxidizing agent, a second oxidizing agent, or both in sequence, wherein the first oxidizing agent may comprise sodium chlorite ($NaClO_2$), and the second oxidizing agent may comprise hydrogen peroxide ($H_2O_2$).

According to an embodiment, the decolorization process may be carried out by the second oxidizing agent.

The amount of hydrogen peroxide used in the decolorization process may be 0.01% by weight or more, 0.02% by weight or more, 0.03% by weight or more, 0.04% by weight or more, 0.05% by weight or more, 0.06% by weight or more, 0.1% by weight or more, 0.2% by weight or more, 0.3% by weight or more, 0.4% by weight or more, or 0.5% by weight or more, and 2.0% by weight or less, 1.7% by weight or less, 1.5% by weight or less, 1.3% by weight or less, 1.2% by weight or less, 1.1% by weight or less, 1% by weight or less, 0.9% by weight or less, 0.8% by weight or less, 0.7% by weight or less, or 0.6% by weight or less, based on the total weight of the crude solution.

The decolorization process may be carried out under basic pH conditions. For example, the pH range in the decolorization may be 7 or more, 7.5 or more, 8 or more, or 8.5 or more, and may be 13 or less, 12 or less, 11 or less, 10.5 or less, 10 or less, 9.5 or less, or 9 or less. As a specific example, the decolorization may be carried out at a pH of 7 to 13 or pH 8 to 13. As a more specific example, the decolorization may be carried out at a pH of 8 to 11 or pH 8 to 10.

An alkaline component may be used to adjust the basic pH. Examples of the alkaline component used for pH adjustment in the decolorization process include sodium hydroxide, potassium hydroxide, lithium hydroxide, and sodium carbonate, but it is not limited thereto. The crude solution can be adjusted to a desired basic pH as it comprises an appropriate amount of such an alkaline component.

In addition, the temperature in the decolorization process may be 40°C or higher, 45°C or higher, 50°C or higher, 55°C or higher, or 60°C or higher, and 90°C or lower, 80°C or lower, 75°C or lower, 70°C or lower, or 65°C or lower. As a specific example, the decolorization may be carried out at a temperature of 40°C to 90°C or 50°C to 70°C.

In addition, the time required for the decolorization process may be 10 minutes or more, 30 minutes or more, or 50 minutes or more, and 3 hours or less, 2 hours or less, or 1 hour and 30 minutes or less. As a specific example, it may be 30 minutes to 1 hour and 30 minutes.

According to another embodiment, the decolorization process may be carried out by the first oxidizing agent and the second oxidizing agent in sequence. Specifically, the decolorization process may sequentially comprise a first decolorization process using the first oxidizing agent and a second decolorization process using the second oxidizing agent.

The first decolorization process may be carried out by a first oxidizing agent comprising sodium chlorite ($NaClO_2$). When decolorization is carried out using sodium chlorite as an oxidizing agent, the effect of color improvement is significant, and there is almost no decrease in molecular weight, unlike other chlorine-based oxidizing agents (such as NaClO). This is attributed to the fact that cell-derived impurities (proteins, lipids, and the like) in the crude solution are decomposed by the reaction with sodium chlorite, whereas the PHA is hardly decomposed.

The amount of sodium chlorite used in the decolorization process may be 0.01% by weight or more, 0.02% by weight or more, 0.03% by weight or more, 0.04% by weight or more, 0.05% by weight or more, 0.06% by weight or more, 0.1% by weight or more, 0.2% by weight or more, 0.3% by weight or more, 0.4% by weight or more, or 0.5% by weight or more, and 1.3% by weight or less, 1.2% by weight or less, 1.1% by weight or less, 1% by weight or less, 0.9% by weight or less, 0.8% by weight or less, 0.7% by weight or less, or 0.6% by weight or less, based on the total weight of the crude solution. As a specific example, in the first decolorization, the sodium chlorite may be used in an amount of 0.01 to 1% by weight or 0.05 to 1% by weight based on the total weight of the crude solution. When the amount of sodium chlorite used is within the above preferred range, the decolorization effect can be sufficiently achieved while the reduction in molecular weight is minimized.

The first decolorization may be carried out under acidic pH conditions. For example, the pH range in the first decolorization may be 7 or less, 6.5 or less, 6 or less, 5.5 or less, 5 or less, 4.5 or less, or 4 or less, and 1 or more, 1.5 or more, 2 or more, 2.5 or more, 3 or more, or 3.5 or more. As a specific example, the first decolorization may be carried out at a pH of 1 to 7 or pH 2 to 7. As a more specific example, the first decolorization may be carried out at a pH of 3 to 6 or pH 3 to 5.

An acid component may be used to adjust the acidic pH. Examples of the acid component used for pH adjustment in the first decolorization include phosphoric acid, sulfuric acid, hydrochloric acid, and nitric acid, but it is not limited thereto. The crude solution can be adjusted to a desired acidic pH as it comprises an appropriate amount of such an acid component.

In addition, the temperature in the first decolorization may be 40°C or higher, 45°C or higher, 50°C or higher, 55°C or higher, or 60°C or higher, and 90°C or lower, 80°C or lower, 75°C or lower, 70°C or lower, or 65°C or lower. As a specific example, the first decolorization may be carried out at a temperature of 40°C to 90°C or 50°C to 70°C.

In addition, the time required for the first decolorization may be 10 minutes or more, 30 minutes or more, or 50 minutes or more, and 3 hours or less, 2 hours or less, or 1 hour and 30 minutes or less. As a specific example, it may be 30 minutes to 1 hour and 30 minutes.

The second decolorization may be carried out with a second oxidizing agent comprising hydrogen peroxide ($H_2O_2$). The pH conditions for the second decolorization and examples of alkaline components added therefor, the amount of oxidizing agent added, and the temperature and time conditions may be the same as those exemplified above in the decolorization process using hydrogen peroxide.

When hydrogen peroxide is used as an oxidizing agent in the second decolorization step once sodium chlorite has been used as an oxidizing agent in the first decolorization step, the content of impurities (proteins and lipids) after the second decolorization can be significantly reduced, and the rate at which impurities are re-adsorbed during the recovery of a solid (PHA) can be reduced. Accordingly, the amount of enzyme used for protein decomposition can be reduced in the subsequent deproteinization step, thereby lowering the production cost. In addition, the amount of hydrogen peroxide used in the second decolorization may be smaller owing to the sequential use with sodium chlorite (first oxidizing agent).

A deproteinization process may be carried out after the decolorization process. The deproteinization process may be carried out by a protease. Examples of the protease include alcalase, pepsin, trypsin, papain, chymotrypsin, aminopeptidase, and carboxypeptidase, one, two, or more of which may be used. The deproteinization may be carried out under basic pH conditions. For example, the pH range in the deproteinization may be 7 or more, 7.5 or more, 8 or more, or 8.5 or more, and may be 12 or less, 11 or less, 10.5 or less, 10 or less, 9.5 or less, or 9 or less. In addition, the temperature in the deproteinization process may be 40°C or higher, 45°C or higher, 50°C or higher, 55°C or higher, or 60°C or higher, and 90°C or lower, 80°C or lower, 75°C or lower, 70°C or lower, or 65°C or lower.

In addition, the supernatant may be separated from the process solution during the purification process, or the supernatant may be separated from the purified solution upon the purification process, to be used for the measurement of a color value, which will be explained in detail in the monitoring of a color value below.

**Control of the purification process conditions through monitoring of color values**

According to an embodiment, the method of the present disclosure comprises measuring and analyzing a color value of a process solution or its supernatant during the purification process to control the conditions of the purification process.

For example, the measurement of the color value comprises measuring the a* value of the process solution or its supernatant in the CIE LAB color space, and the purification process conditions may be adjusted based on the change in the a* value of the process solution or its supernatant.

For example, the change in the a* value may be confirmed by comparing the a* value of the process solution or its supernatant with the a* value of the crude solution or its supernatant before the purification process, or by the change in the a* value of the process solution or its supernatant over time during the purification process.

As a specific example, the change in the a* value of the process solution or its supernatant is monitored at the beginning of the purification process to distinguish normal and abnormal states based on the increase or decrease thereof. If it is abnormal, the amount of an oxidizing agent added to the decolorization process can be increased. For this purpose, the process further comprises measuring the a* value of the crude solution or its supernatant, and the change in the a* value of the process solution or its supernatant relative to the a* value of the crude solution or its supernatant may be confirmed.

As another specific example, the change in the a* value of the process solution or its supernatant is continuously monitored while the purification process is carried out to distinguish normal and abnormal states based on the increase or decrease thereof. If it is abnormal, the amount of an oxidizing agent added to the decolorization process can be increased. For this purpose, after the purification process has been initiated, the a* value of the process solution or its supernatant is repeatedly measured at regular time intervals, for example, at 10-minute intervals, 20-minute intervals, or 30-minute intervals. These a* values may be compared to determine whether there is a decrease or an increase.

According to another embodiment, the method of the present disclosure comprises measuring a color value of a purified solution and its supernatant after the purification process, based on which to predict the color value of a final PHA. If the predicted color value does not meet the target color value, additional purification processes may be carried out.

Specifically, the a* values of the purified solution and its supernatant are monitored after the deproteinization process, from which the b* value of a final PHA resin is predicted to determine suitability. If it is determined to be unsuitable, an additional decolorization process may be carried out.

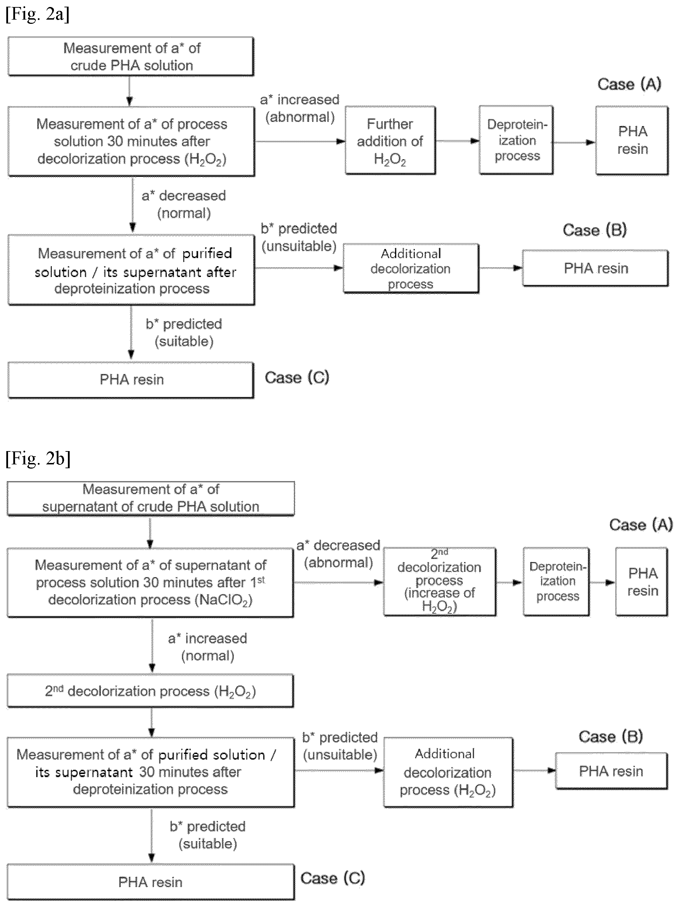

Fig. 2a illustrates a process for preparing a PHA resin comprising a decolorization process (using only hydrogen peroxide) according to an embodiment.

Fig. 2b illustrates a process for preparing a PHA resin comprising a decolorization process (using sodium chlorite or the like) according to another embodiment.

Referring to Figs. 2a and 2b, the purification process for preparing a PHA resin may be divided into cases where (A) the purification process is carried out abnormally, (B) the purification process is carried out normally whereas the color value of a final product is predicted to be unsuitable, and (C) the purification process is carried out normally while the color value of a final product is predicted to be suitable.

In cases (A) and (B), PHA resins with low color quality are obtained; thus, it is necessary to respond appropriately through the monitoring of color values.

**Case (A) where the purification process is carried out abnormally**

If the content of impurities in the initial crude PHA solution is high, the oxidizing agent added in the decolorization process would not remove the impurities attached to the PHA particles; rather, it would first react with the impurities dissolved in the crude solution, resulting in a shortage of the oxidizing agent; thus, the decolorization process is not carried out normally. Therefore, the changes in color values (changes in a* of the process solution) in the initial stage between the normal and abnormal progresses of the decolorization process are different.

According to an embodiment, referring to Fig. 2a, if the a* value of the process solution increases during the decolorization process using the second oxidizing agent ($H_2O_2$) relative to the a* value of the crude solution, it may be viewed as abnormal. Therefore, the amount of the oxidizing agent added may be increased. Specifically, the amount of the oxidizing agent added to the decolorization process may be increased when the following Relationship (1A) is satisfied.

$$a0 < a30 \qquad (1A)$$

In Relationship (1A), a0 is the a* value of the crude solution, and a30 is the a* value of the process solution after the addition of the second oxidizing agent.

The timing at which the a* value of the process solution is measured after the addition of the second oxidizing agent may be, for example, 1 minute, 5 minutes, 10 minutes, 20 minutes, 30 minutes, or 60 minutes after the addition of the second oxidizing agent. Specifically, it may be about 30 minutes after the addition of the second oxidizing agent.

According to another embodiment, referring to Fig. 2b, if the a* value of the supernatant of the process solution decreases during the decolorization process using the first oxidizing agent ($NaClO_2$) relative to the a* value of the crude solution, it may be viewed as abnormal. Therefore, the amount of the oxidizing agent added may be increased. Specifically, the amount of at least one of the first oxidizing agent and the second oxidizing agent added to the decolorization process may be increased, more specifically, the amount of the second oxidizing agent added may be increased, when the following Relationship (1B) is satisfied.

$$a0' > a30' \qquad (1B)$$

In Relationship (1B), a0' is the a* value of the supernatant of the crude solution, and a30' is the a* value of the supernatant of the process solution after the addition of the first oxidizing agent.

The timing at which the a* value of the supernatant of the process solution is measured after the addition of the first oxidizing agent may be, for example, 1 minute, 5 minutes, 10 minutes, 20 minutes, 30 minutes, or 60 minutes after the addition of the first oxidizing agent. Specifically, it may be about 30 minutes after the addition of the first oxidizing agent.

As described above, in the decolorization process using sodium chlorite, the color value of only the supernatant of a process solution is monitored, and the supernatant may be obtained by treating the process solution by centrifugation or the like.

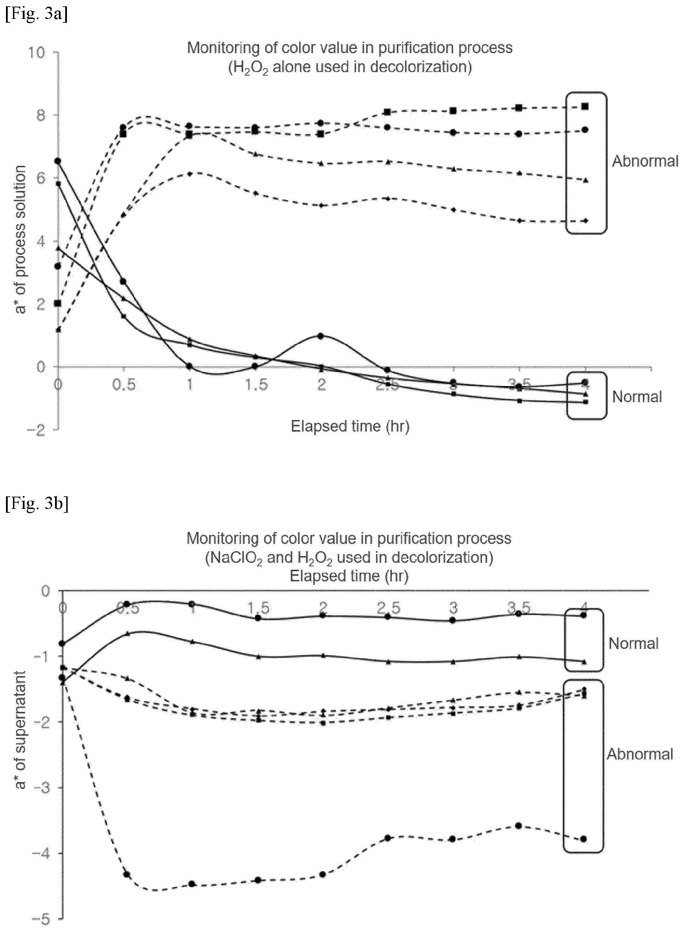

In particular, the determination of the normal and abnormal states based on the change in the a* value of the process solution (or its supernatant) is different in the decolorization process using hydrogen peroxide and the decolorization process using sodium chlorite. Referring to Fig. 3a, if the a* value of the process solution increases 30 minutes after the addition of hydrogen peroxide, it may be determined as abnormal, whereas, referring to Fig. 3b, if the a* value of the supernatant decreases 30 minutes after the addition of sodium chlorite, it may be determined as abnormal.

## Case (B) where the color value of a final product is predicted to be unsuitable

Meanwhile, even if the purification process for a polyhydroxyalkanoate resin is carried out normally, there may be cases where the desired final properties (color values) are not achieved due to reasons such as a rapid decrease in the activity of the oxidizing agent afterward.

In such a case, the color value of the purified solution after the purification process (e.g., decolorization and deproteinization process) is measured, from which the color value of a final PHA resin may be predicted to take appropriate measures in advance.

Accordingly, the method of the present disclosure may comprise monitoring the color value of the purified solution after the purification process to predict the color value of a polyhydroxyalkanoate resin.

Upon the purification process, the impurities attached to the surface of the PHA particles fall off and exist in a dissolved state in the solvent.

When this is taken into account, as shown in Fig. 5, it can be assumed that the color of the PHA resin alone mixed in the purified solution can be predicted by excluding the color of the supernatant in which the impurities are dissolved from the color of the purified solution (PHA and impurities are mixed in the solvent).

Accordingly, the measurement of the color value of the purified solution comprises measuring the a* value of the purified solution and its supernatant in the CIE LAB color space, and the b* value of the polyhydroxyalkanoate resin in the CIE LAB color space may be predicted based on the a* value of the purified solution and its supernatant.

Specifically, the present inventors have measured the a* value of a purified solution obtained through a purification process, the a* value of its supernatant, and the b* value of a final PHA resin in a number of processes, whereby it is possible to derive a correlation through statistical analysis based on these data. The statistical analysis method may be, for example, regression analysis, specifically, linear regression analysis or nonlinear regression analysis, more specifically, multiple linear regression analysis, but it is not limited thereto.

As an example, the b* value of the polyhydroxyalkanoate resin may be predicted from the following Relationship (2A).

$$b* = Y + (X1 \times a1) + (X2 \times a2) \qquad (2A)$$

In Relationship (2A), a1 is the a* value of the purified solution, and a2 is the a* value of the supernatant of the purified solution.

In Relationship (2A), Y may be 10 or more, 11 or more, 12 or more, 13 or more, 14 or more, 15 or more, or 15.5 or more, and may be 21 or less, 20 or less, 19 or less, 18 or less, 17 or less, 16.5 or less, or 16 or less. In Relationship (2A), X1 may be -2.5 or more, -2.0 or more, -1.5 or more, -1 or more, -0.5 or more, 0 or more, 0.3 or more, or 0.5 or more, and may be 4.0 or less, 3.5 or less, 3 or less, 2.5 or less, 2 or less, 1.5 or less, 1 or less, or 0.7 or less. In Relationship (2A), X2 may be -3.5 or more, -3.0 or more, -2.5 or more, -2 or more, -1.5 or more, -1 or more, or -0.5 or more, and may be 3.0 or less, 2.5 or less, 2 or less, 1.5 or less, 1 or less, 0.5 or less, or 0 or less. As a specific example, in Relationship (2A), Y may be 10 to 21 or 14.68 to 16.68, X1 may be -2.5 to 4.0 or -0.39 to 1.61, and X2 may be -3.5 to 3.0 or -1.35 to 0.65. As a more specific example, in Relationship (2A), Y may be about 15.68, X1 may be about 0.61, and X2 may be about -0.35.

As another example, the b* value of the polyhydroxyalkanoate resin may be predicted from the following Relationship (2B).

$$b* = Y' + (X1' \times a1') + (X2' \times a2') \qquad (2B)$$

In Relationship (2B), a1' is the a* value of the purified solution, and a2' is the a* value of the supernatant of the purified solution.

In Relationship (2B), Y' may be 4 or more, 5 or more, 6 or more, 7 or more, 8 or more, 8.5 or more, or 9 or more, and may be 15 or less, 14 or less, 13 or less, 12 or less, 11 or less, 10 or less, or 9.5 or less. In Relationship (2B), X1' may be -7.0 or more, -6.5 or more, -6 or more, -5.5 or more, -5 or more, or -4.5 or more, and may be -1.0 or less, -1.5 or less, -2.0 or less, -2.5 or less, -3.0 or less, -3.5 or less, or -4.0 or less. In Relationship (2B), X2' may be -8 or more, -7 or more, -6 or more, -5.5 or more, -5 or more, -4.5 or more, or -4.3 or more, and may be -1 or less, -1.5 or less, -2 or less, -2.5 or less, -3 or less, -3.5 or less, or -4 or less. As a specific example, in Relationship (2B), Y' may be 4 to 15 or 8.15 to 10.15, X1' may be -7 to -1 or -5.15 to -3.15, and X2' may be -8 to -1 or -5.04 to -3.04. As a more specific example, in Relationship (2B), Y' may be about 9.15, X1' may be about -4.15, and X2' may be about -4.04.

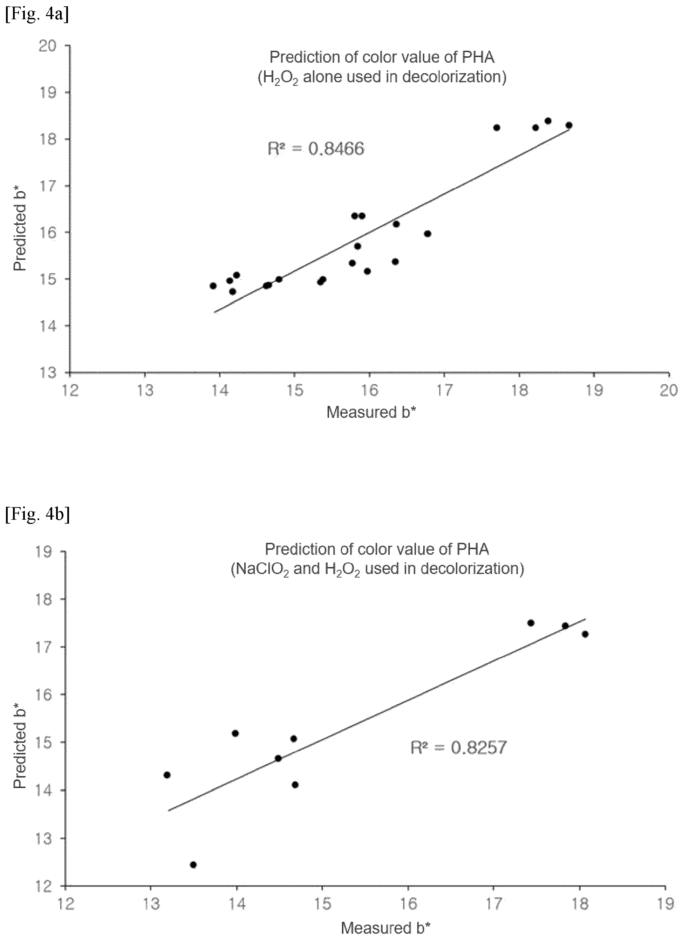

Referring to Figs. 4a and 4b, the measured b* value and the predicted b* value of a PHA resin show a high correlation.

As the b* value of a PHA resin is predicted after the purification process as described above, a further purification process may be carried out prior to the recovery of a solid (PHA) if the target b* range is not met. The target b* value may be, for example, less than 15, specifically, 14 or less, 13 or less, 12 or less, 11 or less, or 10 or less.

Specifically, if the predicted b* value is greater than the target b* value in the preparation process, the purification process may be further carried out, more specifically, the decolorization process may be further carried out. The further decolorization may be carried out once or more, and the specific conditions for the decolorization process may be as exemplified above.

After the purification, the solid is recovered. Water washing and dewatering are carried out to obtain a purified final polyhydroxyalkanoate resin.

**Polyhydroxyalkanoate resin**

The polyhydroxyalkanoate resin prepared according to the process of the present disclosure can have improved color. For example, the polyhydroxyalkanoate resin may have a b* value of 20 or less in the CIE LAB color coordinate. Specifically, the b* value of the polyhydroxyalkanoate resin in the CIE LAB color coordinate may be 14 or less, 13.5 or less, 13 or less, and may be 0 or more, 1 or more, 5 or more, or 10 or more.

The polyhydroxyalkanoate resin may have a glass transition temperature (Tg) of, for example, -45°C to -10°C, -35°C to -10°C, -35°C to -15°C, -35°C to -20°C, or -30°C to -20°C.

In addition, the melting temperature (Tm) of the polyhydroxyalkanoate resin, for example, may not be measured or, for example, may be 100°C to 170°C, 100°C to 160°C, 110°C to 160°C, or 120°C to 150°C.

The polyhydroxyalkanoate resin of the present disclosure may comprise an amorphous polyhydroxyalkanoate, a semi-crystalline polyhydroxyalkanoate, and a mixture thereof. For example, the crystallization temperature (Tc) of the polyhydroxyalkanoate resin may not be measured, or the crystallization temperature (Tc) may be measured to be 60°C to 120°C, specifically, 60°C to 110°C, 70°C to 120°C, or 75°C to 115°C.

The repeat units that constitute the polyhydroxyalkanoate resin may comprise at least one selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxybutyrate (4-HB), 4-hydroxyvalerate (4-HV), 5-hydroxyvalerate (5-HV), and 6-hydroxyhexanoate (6-HH).

According to an embodiment, the polyhydroxyalkanoate resin may comprise a 4-hydroxybutyrate (4-HB) repeat unit. Specifically, it may comprise a copolymer comprising a 4-HB repeat unit. For example, the content of a 4-hydroxybutyrate (4-HB) repeat unit in the polyhydroxyalkanoate resin may be 0.1% by weight or more, 1% by weight or more, 5% by weight or more, 10% by weight or more, 20% by weight or more, 30% by weight or more, 40% by weight or more, 50% by weight or more, or 60% by weight or more, and 100% by weight or less, 99% by weight or less, 90% by weight or less, 80% by weight or less, 70% by weight or less, or 60% by weight or less, based on the total weight of the polyhydroxyalkanoate resin. As a specific example, the polyhydroxyalkanoate resin may comprise a 4-hydroxybutyrate (4-HB) repeat unit in an amount of 25% by weight or more.

In addition, the polyhydroxyalkanoate resin may have a weight average molecular weight (Mw) of, for example, 50,000 or more, 100,000 or more, 200,000 or more, 300,000 or more, 400,000 or more, or 500,000 or more, and may be 1,200,000 or less, 900,000 or less, 800,000 or less, 700,000 or less, or 600,000 or less.

In addition, the polyhydroxyalkanoate resin may have a purity of 95% or more, 97% or more, 98% or more, 98.5% or more, 98.55% or more, or 98.6% or more.

**Mode for the Invention**

Hereinafter, the present disclosure will be described in more detail with reference to embodiments. However, these examples are provided only for illustration purposes, and the present disclosure is not limited thereto.

**Preparation Example 1: Preparation of a PHA fermentation broth**

A genetically engineered *E. coli* strain was used to prepare a PHA fermentation broth. First, the PHA-producing strain was seed cultured to secure a certain amount, and main fermentation was then carried out. For the fermentation of the PHA-producing strain, glucose or raw sugar was used as a carbon source, and various mineral components were added as nutrients. A sodium hydroxide solution or ammonia gas was used to adjust the fermentation pH. The fermentation process using the PHA-producing strain was carried out at 30 to 40°C and a pH of 6 to 9.

**Preparation Example 2: Preparation of a crude PHA solution**

The PHA fermentation broth was subjected to a solid-liquid separation using a mechanical separator to separate the culture medium impurities and biomass containing the PHA. The separated biomass was adjusted to have a total solids content of 10 to 15% by weight. A surfactant (sodium laureth sulfate, 30%, Miwon Corporation) was added in an amount of 0.13% by weight based on the total weight of the reaction solution. Upon sufficient stirring, physical cell crushing was carried out using a high-pressure homogenizer (Bertoli) (400 to 800 bar, 1 pass). The crude PHA solution thus obtained was subjected to a second solid-liquid separation to separate the PHA-containing solids and cell-derived impurities to obtain a crude PHA solution (a solution containing an unpurified PHA).

**Preparation Example 3A: Decolorization process - using hydrogen peroxide alone**

A 20% aqueous solution of sodium hydroxide (NaOH) was added to the crude PHA solution to adjust the pH to 9 to 9.5. A 30% aqueous solution of hydrogen peroxide ($H_2O_2$) was added in an amount of 0.44 to 0.77% by weight based on the total weight of the reaction solution. The decolorization reaction was carried out for 2 hours in a shaking incubator (IS-971R, JEIO TECH) at a temperature of 60°C.

**Preparation Example 3B: Decolorization process - using sodium chlorite and hydrogen peroxide in sequence**

(1) First decolorization process

A 23% aqueous solution of sodium chlorite ($NaClO_2$), whose pH had been adjusted to 6.0 using a 10% aqueous solution of phosphoric acid ($H_3PO_4$), was added in an amount of 0.22 to 0.55% by weight based on the total weight of the crude PHA solution. The final pH of the reaction solution was fixed at 4.0 using a 10% aqueous solution of phosphoric acid. The reaction was carried out for 1 hour in a shaking incubator (IS-971R, JEIO TECH) at a temperature of 60°C.

(2) Second decolorization process

A 20% aqueous solution of sodium hydroxide (NaOH) was added to the first decolorized slurry to adjust the pH to 9 to 9.5. A 30% aqueous solution of hydrogen peroxide was added in an amount of 0.22 to 0.55% by weight based on the total weight of the reaction solution. The decolorization reaction was carried out for 1 hour in a shaking incubator (IS-971R, JEIO TECH) at a temperature of 60°C.

**Preparation Example 4: Deproteinization process**

A 20% aqueous solution of sodium hydroxide was added to the reaction solution obtained by the decolorization process to adjust the pH to 9.5. A protease (Alcalase™) was added in an amount of 0.011 to 0.044% by weight based on the total weight of the reaction solution. The deproteinization reaction was carried out for 2 hours in a shaking incubator (IS-971R, JEIO TECH) at a temperature of 60°C. After the deproteinization, the solid (PHA) recovered was washed with water and then dried to obtain a purified final polyhydroxyalkanoate resin.

**Test Example 1: Derivation of factors reflecting the behavior of impurities between processes (multiple linear regression analysis)**

The three color values (L*, a*, and b*) in the CIE LAB color space were measured for the purified solutions obtained through the purification process (decolorization and deproteinization) under various conditions. The b* value of the PHA resin finally obtained from the purified solution was measured. A spectrophotometer (CM-5, Konica Minolta) was used for the measurements. Since the b* value of a final PHA resin increases as the amount of impurities increases, the b* value reflects the content of impurities in the final PHA resin to some extent.

It was analyzed which of the three color values of the purified solution, alone or in combination of two color values thereof, had a significant correlation with the color value of the final product (i.e., the effect of impurities removal). The three color values (L*, a*, and b*) of the purified solution obtained from 67 different purification processes were measured. The b* value of the PHA resin finally obtained from each process was subjected to multiple linear regression analysis (n = 67). The results are shown in Table 1 below.

[Table 1]

| | Multiple linear regression analysis (n = 67) - b* of the PHA resin | | | |
|---|---|---|---|---|
| | βi coefficient | Standard error | t value | p value |
| Intercept | 12.0446 | 3.4128 | 3.5292 | $7.8 \times 10^{-4}$ |
| x1 (L* of the purified solution) | 0.0370 | 0.1334 | 0.2777 | 0.7821 |
| x2 (a* of the purified solution) | 0.7781 | 0.1926 | 4.0408 | $1.5 \times 10^{-4}$ |
| x3 (b* of the purified solution) | 0.1470 | 0.1048 | 1.4030 | 0.1655 |

As can be seen from the table above, when the p value, which is an indicator that determines the reliability of a correlation in multiple linear regression analysis, is taken into account, the a* value among the color values of the purified solution upon the purification process had the most significant relationship with the b* value of the final PHA resin.

**Example 1A: Monitoring of color values (using hydrogen peroxide alone)**

Color values were monitored in the purification process, comprising a decolorization process using hydrogen peroxide alone. Specifically, while the preparation of the PHA fermentation broth (Preparation Example 1), the preparation of the crude PHA solution (Preparation Example 2), and the decolorization process using hydrogen peroxide alone (Preparation Example 3A) were carried out, the color values of the process solutions were monitored. Monitoring of the color value was carried out by extracting 12 ml of each process solution, charging it to a 20-mm quartz cell, and measuring the a* value of the transmitted color of the sample.
First, the a* value of the crude PHA solution obtained in Preparation Example 2 (PHA solution recovered from the PHA fermentation broth through cell crushing and washing and before pH adjustment and decolorization) was obtained. Thereafter, while the decolorization process of Preparation Example 3A and the deproteinization process of Preparation Example 4 were carried out, the process solution was sampled at 30-minute intervals to obtain the a* value.
The a* value of the process solution measured 30 minutes after the initiation of the decolorization process using hydrogen peroxide (addition of hydrogen peroxide) was classified as normal progress if it decreased relative to the a* value of the crude PHA solution before the decolorization process, and it was classified as abnormal progress if it increased.
The results are shown in Table 2 below and Fig. 3a.

[Table 2]

| Process | Elapsed time (h) | a* of the process solution - normal progress (a* decreased after 30 minutes) | | | a* of the process solution - abnormal progress (a* increased after 30 minutes) | | | |
|---|---|---|---|---|---|---|---|---|
| Crude PHA solution | 0.0 | 3.76 | 5.80 | 6.50 | 3.18 | 1.99 | 1.16 | 1.16 |
| Decolorization | 0.5 | 2.17 | 1.60 | 2.71 | 7.61 | 7.39 | 4.87 | 4.81 |
| | 1.0 | 0.87 | 0.69 | - | 7.62 | 7.39 | 7.35 | 6.14 |
| | 1.5 | 0.34 | 0.29 | - | 7.60 | 7.45 | 6.76 | 5.50 |
| | 2.0 | -0.07 | 0.02 | 0.98 | 7.74 | 7.39 | 6.47 | 5.13 |
| Deproteinization | 2.5 | -0.36 | -0.55 | -0.12 | 7.59 | 8.07 | 6.53 | 5.36 |
| | 3.0 | -0.54 | -0.88 | -0.53 | 7.44 | 8.12 | 6.30 | 5.00 |
| | 3.5 | -0.69 | -1.07 | -0.64 | 7.40 | 8.21 | 6.15 | 4.65 |
| | 4.0 | -0.87 | -1.14 | -0.52 | 7.49 | 8.24 | 5.95 | 4.63 |

As can be seen from Table 2 above and Fig. 3a, the a* values of the initial crude PHA solution were measured to be 0 to 7. The a* values of the process solution decreased by 40 to 80% relative to the initial values after 30 minutes in the normal progress of the decolorization process, whereas the a* values of the process solution increased by 2.0 to 4.5 times relative to the initial values after 30 minutes in abnormal progress.

**Example 1B: Monitoring of color values (using sodium chlorite and hydrogen peroxide)**

Color values were monitored in the purification process, comprising a decolorization process using sodium chlorite and hydrogen peroxide. Specifically, while the preparation of the PHA fermentation broth (Preparation Example 1), the preparation of the crude PHA solution (Preparation Example 2), and the decolorization process using sodium chlorite and hydrogen peroxide in sequence (Preparation Example 3B) were carried out, the process solutions was centrifuged to obtain its supernatant, and the color values thereof were monitored.

Monitoring of the color values was carried out by extracting 15 ml of each process solution, which was centrifuged in a centrifuge (Combi-514R, Hanil) at 3,800 rpm for 20 minutes, charging 12 ml of the supernatant thus separated to a 20-mm quartz cell, and measuring the a* value of the transmitted color of the sample.

First, the a* value of the supernatant of the crude PHA solution obtained in Preparation Example 2 (PHA solution recovered from the PHA fermentation broth through cell crushing and washing and before pH adjustment and decolorization) was obtained. Thereafter, while the first and second decolorization processes of Preparation Example 3B and the deproteinization process of Preparation Example 4 were carried out, the process solution was sampled at 30-minute intervals, and the supernatants thereof were measured for the a* value.

The a* value of the supernatant of the process solution measured 30 minutes after the initiation of the first decolorization process using sodium chlorite (addition of sodium chlorite) was classified as normal progress if it increased relative to the a* value of the supernatant of the crude PHA solution before the decolorization process, and it was classified as abnormal progress if it decreased.

The results are shown in Table 3 below and Fig. 3b.

[Table 3]

| Process | Elapsed time (h) | a* of the supernatant - normal progress (a* increased after 30 minutes) | | a* of the supernatant - abnormal progress (a* decreased after 30 minutes) | | | |
|---|---|---|---|---|---|---|---|
| Crude PHA solution | 0.0 | -1.39 | -0.81 | -1.33 | -1.17 | -1.17 | -1.17 |
| First decolorization | 0.5 | -0.66 | -0.22 | -4.32 | -1.34 | -1.66 | -1.63 |
| | 1.0 | -0.78 | -0.21 | -4.48 | -1.86 | -1.90 | -1.80 |
| Second decolorization | 1.5 | -1.00 | -0.43 | -4.41 | -1.82 | -1.98 | -1.91 |
| | 2.0 | -0.99 | -0.39 | -4.32 | -1.90 | -2.01 | -1.84 |
| Deproteinization | 2.5 | -1.08 | -0.41 | -3.77 | - | - | - |
| | 3.0 | -1.08 | -0.46 | -3.79 | - | - | - |
| | 3.5 | -1.01 | -0.36 | -3.59 | -1.55 | -1.79 | -1.75 |
| | 4.0 | -1.08 | -0.39 | -3.80 | -1.60 | -1.57 | -1.51 |

As can be seen from Table 3 above and Fig. 3b, the a* values of the supernatant of the initial crude PHA solution were measured to be -2 to 0. The a* values of the supernatant of the process solution increased by 50 to 75% relative to the initial values after 30 minutes in the normal progress of the decolorization process, whereas the a* values of the supernatant of the process solution decreased by 3.5 times relative to the initial value after 30 minutes in abnormal progress.

**Example 2A: Prediction of the b* value of a PHA resin (using hydrogen peroxide alone)**

The color value (a* value) after purification, comprising a decolorization process using hydrogen peroxide alone, was measured to predict the color value (b* value) of a final polyhydroxyalkanoate resin. Specifically, the preparation of the PHA fermentation broth (Preparation Example 1), the preparation of the crude PHA solution (Preparation Example 2), the decolorization process using hydrogen peroxide alone (Preparation Example 3A), and the deproteinization process (Preparation Example 4) were carried out to obtain a purified solution. Its color value was measured, and the final color value was predicted based thereon. The color value of the PHA resin finally obtained through the recovery of a solid was measured and compared with the predicted value.

First, the purified solution was extracted after the deproteinization process, and the a* value was measured. The purified solution was centrifuged to obtain a supernatant, the a* value was measured. Thereafter, the b* value of the polyhydroxyalkanoate resin finally obtained through the recovery of a solid was measured. The measurements of color values were carried out on 21 samples. The results are shown in the table below.

The following relationship was derived through multiple linear regression analysis using the measured color value data.

$$b^* \text{ of final PHA resin} = 15.68 + (0.61 \times a1) + (-0.35 \times a2)$$

Here, a1 is the a* value of the purified solution in the CIE LAB color space, and a2 is the a* value of the supernatant of the purified solution.

In addition, the a* values of the purified solution and the supernatant were substituted into the above relationship to calculate the predicted b* value of the final PHA resin, which are also shown in the table below.

[Table 4]

| Sample | Purified solution upon the purification process (decolorization and deproteinization processes) | | PHA resin | |
| --- | --- | --- | --- | --- |
| | a* of the purified solution | a* of the supernatant of the purified solution | Measured b* | Predicted b* |
| 1 | -0.14 | 0.73 | 15.77 | 15.33 |
| 2 | -0.84 | 0.88 | 14.66 | 14.85 |
| 3 | -1.27 | -0.16 | 14.15 | 14.95 |
| 4 | -0.81 | 0.52 | 14.80 | 14.99 |
| 5 | 0.56 | -0.89 | 15.82 | 16.33 |
| 6 | -0.48 | -0.85 | 15.85 | 15.68 |
| 7 | -1.23 | -0.69 | 15.99 | 15.16 |
| 8 | -1.54 | -0.51 | 15.35 | 14.91 |
| 9 | -1.54 | -0.24 | 13.93 | 14.82 |
| 10 | -1.69 | -0.23 | 14.18 | 14.72 |
| 11 | 4.03 | -0.23 | 17.72 | 18.23 |
| 12 | 3.97 | -0.27 | 18.23 | 18.21 |
| 13 | -1.47 | -0.19 | 14.62 | 14.84 |
| 14 | -1.27 | -0.26 | 15.38 | 14.99 |
| 15 | 4.08 | -0.3 | 18.67 | 18.28 |
| 16 | 4.29 | -0.2 | 18.39 | 18.38 |
| 17 | 0.56 | -0.89 | 15.90 | 16.33 |
| 18 | 0.81 | 0.01 | 16.38 | 16.17 |
| 19 | -0.37 | 0.25 | 16.36 | 15.36 |
| 20 | 0.74 | 0.51 | 16.78 | 15.95 |
| 21 | -0.28 | 1.24 | 14.23 | 15.07 |

The correlation between the measured b* values and the predicted b* values of the PHA resin is shown in Fig. 4a based on the above results, and it is confirmed that the coefficient of determination ($R^2$) is high at 0.8466.

**Example 2B: Prediction of the b* value of a PHA resin (using sodium chlorite and hydrogen peroxide)**

The color value (a* value) after purification, comprising a decolorization process using sodium chlorite and hydrogen peroxide in sequence, was measured to predict the color value (b* value) of a final polyhydroxyalkanoate resin. Specifically, the preparation of the PHA fermentation broth (Preparation Example 1), the preparation of the crude PHA solution (Preparation Example 2), the decolorization process using sodium chlorite and hydrogen peroxide in sequence (Preparation Example 3B), and the deproteinization process (Preparation Example 4) were carried out to obtain a purified solution. Its color value was measured, and the final color value was predicted based thereon. The color value of the polyhydroxyalkanoate resin finally obtained through the recovery of a solid was measured and compared with the predicted value.

First, the purified solution was extracted after the deproteinization process, and the a* value was measured. The purified solution was centrifuged to obtain a supernatant, the a* value was measured. In addition, thereafter, the b* value of the polyhydroxyalkanoate resin finally obtained through the recovery of a solid was measured. The measurements of color values were carried out on 9 samples. The results are shown in the table below.

The following relationship was derived through multiple linear regression analysis using the measured color value data.

$$\text{b* of final PHA resin} = 9.15 + (-4.15 \times a1') + (-4.04 \times a2')$$

Here, a1' is the a* value of the purified solution in the CIE LAB color space, and a2' is the a* value of the supernatant of the purified solution.

In addition, the a* values of the purified solution and the supernatant thereof were substituted into the above relationship to calculate the predicted b* value of the final PHA resin, which are also shown in the table below.

[Table 5]

| Sample | Purified solution upon the purification process (decolorization and deproteinization processes) | | PHA resin | |
| --- | --- | --- | --- | --- |
| | a* of the purified solution | a* of the supernatant of the purified solution | Measured b* | Predicted b* |
| 1 | -0.34 | -1.01 | 14.50 | 14.65 |
| 2 | 0.03 | -0.84 | 13.50 | 12.43 |
| 3 | -0.47 | -0.79 | 13.20 | 14.30 |
| 4 | -0.85 | -0.35 | 14.69 | 14.10 |
| 5 | -1.19 | -0.24 | 14.67 | 15.07 |
| 6 | -1.13 | -0.33 | 13.99 | 15.18 |
| 7 | -1.61 | -0.39 | 17.85 | 17.42 |
| 8 | -1.64 | -0.32 | 18.07 | 17.26 |
| 9 | -1.61 | -0.41 | 17.44 | 17.50 |

The correlation between the measured b* values and the predicted b* values of the PHA resin is shown in Fig. 4b based on the above results, and it is confirmed that the coefficient of determination ($R^2$) is high at 0.8257.

**Example 3: Control of the purification process through monitoring of color values**

When the decolorization process was carried out abnormally in Example 2A, the decolorization process was carried out by increasing the amount of hydrogen peroxide added (1.5 times, 2.0 times). Thereafter, the b* value of the PHA resin finally obtained through the deproteinization process and the recovery of a solid was measured.

In addition, when the decolorization process was carried out abnormally in Example 2A, a decolorization process was further carried out after the deproteinization. Thereafter, the b* value of the PHA resin finally obtained through the recovery of a solid was measured.

In addition, when the decolorization process was carried out abnormally in Example 2B, the decolorization process was carried out by increasing the amount of sodium chlorite added (0.5%). Thereafter, the b* value of the PHA resin finally obtained through the deproteinization process and the recovery of a solid was measured.

In addition, when the decolorization process was carried out abnormally in Example 2B, the decolorization process was carried out by increasing the amount of hydrogen peroxide added (1.5%). Thereafter, the b* value of the PHA resin finally obtained through the deproteinization process and the recovery of a solid was measured.

**Comparative Example**

The purification process was carried out in the same procedure as in Example 3, except that even when the decolorization process was carried out abnormally, its process conditions were not adjusted. Thereafter, the predicted b* values were calculated based on the relationship derived in Example 2A or 2B.

The b* values obtained in the Comparative Example and Example 3 are compared in Table 6 below.

[Table 6]

| Oxidizing agent | Process conditions | Predicted b* in Comparative Example | Measured b* in Example 3 | Reduction rate in b* (%) |
|---|---|---|---|---|
| Hydrogen peroxide alone used | Increased hydrogen peroxide (1.5 times) | 21.17 | 17.65 | 16.63 |
| | Increased hydrogen peroxide (2.0 times) | 20.46 | 17.93 | 12.38 |
| | Additional decolorization after deproteinization | 15.50 | 14.59 | 5.85 |
| Sodium chlorite and hydrogen peroxide used | Increased sodium chlorite (0.5%) | 16.73 | 16.10 | 3.77 |
| | Increased hydrogen peroxide (1.5%) | 16.73 | 15.35 | 8.25 |

As can be seen from Table 6 above, when the decolorization process was carried out abnormally, the b* value of the final PHA resin could be reduced if the amount of oxidizing agent was increased, or additional decolorization was carried out. In particular, increasing the amount of oxidizing agent was more effective in reducing the b* value than carrying out additional decolorization.

**Claims**

1. A method for controlling a purification process, which comprises measuring a color value of a process solution or its supernatant in the purification process for preparing a polyhydroxyalkanoate (PHA) resin, or a color value of a purified solution and its supernatant after the purification process; and controlling the purification process based on the measured color value.

2. The method for controlling a purification process of claim 1, wherein the measurement of the color value comprises measuring the a* value in the CIE LAB color space, and the purification process conditions are adjusted based on the change in the a* value of the process solution or its supernatant.

3. The method for controlling a purification process of claim 2, wherein the change in the a* value is confirmed by comparing the a* value of the process solution or its supernatant with the a* value of a crude solution or its supernatant before the purification process, or by the change in the a* value of the process solution or its supernatant over time during the purification process.

4. The method for controlling a purification process of claim 1, wherein the measurement of the color value comprises measuring an a* value in the CIE LAB color space, and the b* value of the polyhydroxyalkanoate resin in the CIE LAB color space is predicted based on the a* value of the purified solution and its supernatant.

5. A system for controlling a purification process, which comprises a purification unit for carrying out the purification process for preparing a polyhydroxyalkanoate (PHA) resin;

   a monitoring unit for measuring a color value of a process solution or its supernatant during the purification process, or a color value of a purified solution and its supernatant after the purification process, and a control unit for controlling the purification process based on the measured color value.

6. A process for preparing a polyhydroxyalkanoate resin from a crude solution comprising a polyhydroxyalkanoate (PHA) through a purification process, which comprises at least one step among (a) measuring a color value of a process solution or its supernatant during the purification process; and (b) measuring a color value of a purified solution and its supernatant after the purification process.

7. The process for preparing a polyhydroxyalkanoate resin of claim 6, wherein the measurement of the color value in step (a) comprises measuring the a* value of the process solution or its supernatant in the CIE LAB color space, and the purification process conditions are adjusted based on the change in the a* value of the process solution or its supernatant.

8. The process for preparing a polyhydroxyalkanoate resin of claim 7, wherein the process further comprises measuring the a* value of the crude solution or its supernatant, and the change in the a* value of the process solution or its supernatant relative to the a* value of the crude solution or its supernatant is confirmed.

9. The process for preparing a polyhydroxyalkanoate resin of claim 8, wherein the purification process comprises a decolorization process, the decolorization process is carried out by using a first oxidizing agent, a second oxidizing agent, or both **in** sequence, the first oxidizing agent comprises sodium chlorite ($NaClO_2$), and the second oxidizing agent comprises hydrogen peroxide ($H_2O_2$).

10. The process for preparing a polyhydroxyalkanoate resin of claim 9, wherein the decolorization process is carried out by the second oxidizing agent, and

the amount of the oxidizing agent added to the decolorization process is increased when the following Relationship (1A) is satisfied:

$$a0 < a30 \qquad (1A)$$

in Relationship (1A), a0 is the a* value of the crude solution, and a30 is the a* value of the process solution after the addition of the second oxidizing agent.

11. The process for preparing a polyhydroxyalkanoate resin of claim 9, wherein the decolorization process is carried out by the first oxidizing agent and the second oxidizing agent in sequence, and

the amount of at least one of the first oxidizing agent and the second oxidizing agent added to the decolorization process is increased when the following Relationship (1B) is satisfied:

$$a0' > a30' \qquad (1B)$$

in Relationship (1B), a0' is the a* value of the supernatant of the crude solution, and a30' is the a* value of the supernatant of the process solution after the addition of the first oxidizing agent.

12. The process for preparing a polyhydroxyalkanoate resin of claim 6, wherein the measurement of the color value in step (b) comprises measuring the a* value of the purified solution and its supernatant, and the b* value of the polyhydroxyalkanoate resin in the CIE LAB color space is predicted based on the a* value of the purified solution and its supernatant.

13. The process for preparing a polyhydroxyalkanoate resin of claim 12, wherein the b* value of the polyhydroxyalkanoate resin is predicted from the following Relationship (2A):

$$b* = Y + (X1 \times a1) + (X2 \times a2) \qquad (2A)$$

in Relationship (2A), a1 is the a* value of the purified solution, a2 is the a* value of the supernatant of the purified solution, Y is 10 to 21, X1 is -2.5 to 4.0, and X2 is -3.5 to 3.0.

14. The process for preparing a polyhydroxyalkanoate resin of claim 12, wherein the b* value of the polyhydroxyalkanoate resin is predicted from the following Relationship (2B):

$$b* = Y' + (X1' \times a1') + (X2' \times a2') \qquad (2B)$$

in Relationship (2B), a1' is the a* value of the purified solution, a2' is the a* value of the supernatant of the purified solution, Y' is 4 to 15, X1' is -7 to -1, and X2' is -8 to - 1.

**15.** The process for preparing a polyhydroxyalkanoate resin of claim 12, wherein if the predicted b* value of the polyhydroxyalkanoate resin is greater than the target b* value, an additional purification process is carried out.

**16.** The process for preparing a polyhydroxyalkanoate resin of claim 6, wherein the crude solution comprising the polyhydroxyalkanoate is prepared by a method comprising:

performing a solid-liquid separation of a fermentation broth comprising a polyhydroxyalkanoate to obtain biomass;
mixing an additive comprising a surfactant with the biomass to obtain a suspension;
crushing the biomass in the suspension to obtain a slurry; and
performing a solid-liquid separation of the slurry.

[Fig. 1]

[Fig. 2a]

[Fig. 2b]

[Fig. 3a]

Monitoring of color value in purification process
($H_2O_2$ alone used in decolorization)

[Fig. 3b]

Monitoring of color value in purification process
($NaClO_2$ and $H_2O_2$ used in decolorization)
Elapsed time (hr)

[Fig. 4a]

Prediction of color value of PHA
($H_2O_2$ alone used in decolorization)

$R^2 = 0.8466$

[Fig. 4b]

Prediction of color value of PHA
($NaClO_2$ and $H_2O_2$ used in decolorization)

$R^2 = 0.8257$

[Fig. 5]

Color of purified solution (mixture) — Color of supernatant = Color of solid

[Fig. 6]

CIE L*a*b

White L*

Yellow +b*

Green -a*

Red +a*

Blue -b*

Black

## INTERNATIONAL SEARCH REPORT

| International application No. |
|---|
| **PCT/KR2023/011343** |

### A. CLASSIFICATION OF SUBJECT MATTER

**C12P 7/625**(2022.01)i; **C12N 1/20**(2006.01)i

According to International Patent Classification (IPC) or to both national classification and IPC

### B. FIELDS SEARCHED

Minimum documentation searched (classification system followed by classification symbols)

C12P 7/625(2022.01); C07C 29/74(2006.01); C07C 31/18(2006.01); C08G 63/02(2006.01); C08G 63/90(2006.01); C08G 64/00(2006.01); C12P 7/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리하이드록시알카노에이트(polyhydroxyalkanoate), 정제(purification), 모니터링(monitoring), 색가(color value), CIE LAB, L*, a*, b*, 색 공간(color space), 탈색(decolorization), 조액(crude solution)

### C. DOCUMENTS CONSIDERED TO BE RELEVANT

| Category* | Citation of document, with indication, where appropriate, of the relevant passages | Relevant to claim No. |
|---|---|---|
| A | KR 10-2006-0134005 A (PHB INDUSTRIAL S.A.) 27 December 2006 (2006-12-27)<br>See entire document. | 1-16 |
| A | US 7514525 B2 (YU, J.) 07 April 2009 (2009-04-07)<br>See entire document. | 1-16 |
| A | JP 2002-510747 A (METABOLIX, INC.) 09 April 2002 (2002-04-09)<br>See entire document. | 1-16 |
| A | PAGLIANO, G. et al. Recovery of polyhydroxyalkanoates from single and mixed microbial cultures: A review. Frontiers in Bioengineering and Biotechnology. 10 February 2021, vol. 9, 624021, pp. 1-28.<br>See entire document. | 1-16 |
| A | PORRAS, M. A. et al. Novel spectrophotometric technique for rapid determination of extractable PHA using Sudan black dye. Journal of Biotechnology. (electronic publication) 19 June 2017, vol. 255, pp. 28-32.<br>See entire document. | 1-16 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **10 November 2023** | **10 November 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/011343**

### C.    DOCUMENTS CONSIDERED TO BE RELEVANT

| Category* | Citation of document, with indication, where appropriate, of the relevant passages | Relevant to claim No. |
|---|---|---|
| A | US 7919658 B2 (ADKESSON, D. M. et al.) 05 April 2011 (2011-04-05)<br>See entire document. | 1-16 |

Form PCT/ISA/210 (second sheet) (July 2022)

## INTERNATIONAL SEARCH REPORT
### Information on patent family members

International application No.

**PCT/KR2023/011343**

| Patent document cited in search report | | | Publication date (day/month/year) | Patent family member(s) | | | Publication date (day/month/year) |
|---|---|---|---|---|---|---|---|
| KR | 10-2006-0134005 | A | 27 December 2006 | AU | 2004-293501 | A1 | 09 June 2005 |
| | | | | AU | 2004-293501 | B2 | 29 October 2009 |
| | | | | CA | 2547689 | A1 | 09 June 2005 |
| | | | | CA | 2547689 | C | 21 October 2014 |
| | | | | CN | 1886517 | A | 27 December 2006 |
| | | | | CN | 1886517 | B | 17 July 2013 |
| | | | | CN | 1886517 | C | 27 December 2006 |
| | | | | EP | 1687436 | A2 | 09 August 2006 |
| | | | | EP | 1687436 | B1 | 07 January 2009 |
| | | | | JP | 2007-512007 | A | 17 May 2007 |
| | | | | JP | 4709766 | B2 | 22 June 2011 |
| | | | | US | 2007-0161096 | A1 | 12 July 2007 |
| | | | | US | 9045595 | B2 | 02 June 2015 |
| | | | | WO | 2005-052175 | A2 | 09 June 2005 |
| | | | | WO | 2005-052175 | A3 | 30 June 2005 |
| US | 7514525 | B2 | 07 April 2009 | US | 2008-220505 | A1 | 11 September 2008 |
| JP | 2002-510747 | A | 09 April 2002 | AU | 1999-34808 | A1 | 25 October 1999 |
| | | | | AU | 1999-34808 | B2 | 27 February 2003 |
| | | | | CA | 2327086 | A1 | 14 October 1999 |
| | | | | EP | 1070135 | A1 | 24 January 2001 |
| | | | | EP | 1070135 | B1 | 24 February 2010 |
| | | | | US | 2001-0006802 | A1 | 05 July 2001 |
| | | | | US | 6368836 | B2 | 09 April 2002 |
| | | | | WO | 99-51760 | A1 | 14 October 1999 |
| US | 7919658 | B2 | 05 April 2011 | CA | 2522928 | A1 | 25 November 2004 |
| | | | | CA | 2522928 | C | 24 September 2013 |
| | | | | CN | 1816629 | A | 09 August 2006 |
| | | | | CN | 1816629 | C | 09 August 2006 |
| | | | | EP | 1625224 | A2 | 15 February 2006 |
| | | | | EP | 1625224 | B1 | 28 July 2010 |
| | | | | EP | 2239334 | A1 | 13 October 2010 |
| | | | | JP | 2007-502325 | A | 08 February 2007 |
| | | | | JP | 2011-126911 | A | 30 June 2011 |
| | | | | JP | 4814794 | B2 | 16 November 2011 |
| | | | | KR | 10-1105957 | B1 | 18 January 2012 |
| | | | | KR | 10-2006-0052677 | A | 19 May 2006 |
| | | | | TW | 200506063 | A | 16 February 2005 |
| | | | | US | 2005-0069997 | A1 | 31 March 2005 |
| | | | | US | 2011-0152581 | A1 | 23 June 2011 |
| | | | | US | 2011-0152583 | A1 | 23 June 2011 |
| | | | | US | 8183417 | B2 | 22 May 2012 |
| | | | | WO | 2004-101479 | A2 | 25 November 2004 |
| | | | | WO | 2004-101479 | A3 | 03 February 2005 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20010009719